# EUROPEAN PATENT APPLICATION

(11) **EP 3 124 057 A1**
(43) Date of publication of application: **01.02.2017**
(21) Application number: 14887587.5
(22) Date of filing: 14.10.2014
(51) Int. Cl.: A61L 27/12, A61C 8/00

(54) **CALCIUM PHOSPHATE-BASED BIODEGRADABLE MICRO-SCREW AND MANUFACTURING METHOD THEREFOR**

(30) Priority: 27.03.2014 KR 20140036316
(71) Applicant: Kuwotech Co. Ltd., Gwangju 500-460 (KR)
(72) Inventor: JEONG, Cheol woong, Gwangju 502-886 (KR); JUNG, Chul, Gwangju 506-738 (KR); KIM, Min, Gwangju 503-807 (KR); SHIN, Jin Wook, Jeonju-si Jeollabuk-do 560-723 (KR); HONG, Ju hyun, Gwangju 506-824 (KR)
(74) Representative: Zech, Stefan Markus
(86) International application number: PCT/KR2014/009636
(87) International publication number: WO 2015/147396

(57) **Abstract**

Disclosed is a method of manufacturing a calcium phosphate-based bioabsorbable microscrew for fixing a barrier membrane that protects alveolar material used in guided bone regeneration, which is performed to reinforce alveolar material absorbed into an implantation site for a dental implant procedure and to repair a local bone defect, the method including mixing a hydroxyapatite powder with a β-tricalcium phosphate powder to prepare a composite powder, adding and mixing the composite powder with a binder to prepare a screw-molding powder, molding the screw-molding powder into a microscrew using an injection-molding machine, primarily heat-treating the microscrew, and secondarily heat-treating the primarily heat-treated microscrew. The microscrew thus manufactured can be bioabsorbed, thereby obviating additional surgical removal after healing, preventing a problem of a decreased repair rate due to the excessive metal fixed to a wounded portion and consequent overprotection, and generating no side effects.

## Description

### Technical Field

The present invention relates to a calcium phosphate-based bioabsorbable microscrew for fixing a barrier membrane that protects alveolar material used in guided bone regeneration, which is performed to reinforce alveolar material absorbed into an implantation site for a dental implant procedure and to repair a local bone defect, and to a method of manufacturing the same.

### Background Art

Among techniques for installing a replacement tooth in the human body, technology associated with implants is being rapidly developed these days, and the term "implant" originally means an alternative that restores the lost tissue of the human body but has come to indicate the grafting of artificial teeth in the dental field.

An implant process is performed in such a manner that an artificial fixture, which replaces the lost tooth and is made of a material that is not rejected by the human body, is placed in and adhered to the alveolar bone where the tooth is missing, after which the artificial tooth is fixed so as to restore the original function of the tooth.

As such an implant (artificial tooth implantation) process is widely carried out, there are many cases where fixed prosthesis treatment is mainly applied to patients in need of teeth. Furthermore, bone grafting before implantation is required in the case of a wide range of alveolar bone defects, in which initial fixing in the remaining bone cannot be achieved upon implantation, or in the case where the implant cannot be installed at the ideal position or prosthetically modifiable position in the subsequent prosthesis treatment.

In this case, GBR (Guided Bone Regeneration) is performed so that spaces are formed around the bone having insufficient height and width and thus differentiation and migration of cells having bone formability are induced in the remaining bone tissue, thereby reinforcing the alveolar material absorbed into the implantation site and aiding in the repair of local bone defects.

To repair bone defects, autogenous bone and other grafting materials are used. Among grafting materials, the use of autogenous bone is reported to be the best, but is problematic because fixture absorption and bone donation are difficult, the desired amount thereof is not easy to obtain and treatment results are unpredictable. Currently, materials for heterogeneous bones and synthetic bones are being developed and utilized.

However, the use of heterogeneous bone tends to be avoided owing to the risk of infection with specific diseases afflicting bone donor animals. To alleviate the risk, thorough research into synthetic bones is ongoing.

Synthetic bone is exemplified by hydroxyapatite (HA) and β-tricalcium phosphate.

Hydroxyapatite is the same as the main ingredient of bones and teeth in the human body, and has high biocompatibility, and thus an implant in the bone or skin is very compatible with the surrounding biological tissue, but is highly fragile and has weak brittleness and breaking strength and slow re-absorption. Hence, in order to accelerate the functionality and re-absorption thereof, it is used in a mixture with another ceramic material, namely β-tricalcium phosphate, and autogenous bone.

Porous hydroxyapatite, having low density, has a very large surface area, thereby facilitating angiogenesis and bone infiltration. When a sintered body is densely manufactured in the synthesis process, high strength and high compatibility with bone may result, but the use thereof is limited because brittleness is high and osteoconduction and absorption are difficult.

Calcium phosphate-based β-tricalcium phosphate is effective in bone tissue regeneration due to the similarity of the chemical structure to bone minerals, and has effective osteoconductivity due to its porosity that makes it suitable for promoting interconnection of osteoinduction cells.

In particular, synthetic ceramic materials, having biocompatibility and biodegradability, have been employed in various fields including oral and maxillofacial surgery, but they are very fragile and have low mechanical strength.

The aforementioned bone-grafting material is applied to the bone defect site and then barrier membrane treatment is performed. As such, the barrier membrane functions to prevent blood clots and to block the movement of soft tissue (epithelial and connective tissue) cells to the bone defect site, and aids in appropriately positioning bone cells.

The movement of the barrier membrane results in poor bone regeneration because it increases the risk of blood clots and lower bone-grafting materials, and thus, microscrews made of stainless steel or titanium are useful.

When treatment is performed using microscrews, good effects may be obtained by virtue of reduced movement of bone-grafting materials due to the migration of the upper valve, but it is difficult to apply and remove such microscrews. In the case of products using microscrews made of metal alloys, additional surgical removal is required after healing, and material corrosion may often occur. Furthermore, metal, having excessively high strength, is fixed to a wounded portion and thus the wounded portion is overprotected, undesirably resulting in a decreased repair rate.

With the goal of solving the above problems, bioabsorbable polymer materials are currently widely useful for fixing parts, and examples thereof may include PLLA (Polylactic acid), PLGA (Poly(L-lactide-co-glycolide), and PGA (Polyglycolic acid).

Although such materials have high processability, they are too soft and thus have low strength and difficulty in controlling the rate of biodegradation, and do not cause osteoconduction. Such polymers also carry the risk of inducing a non-specific inflammatory response due to an osteolytic reaction.

### Disclosure

### Technical Problem

Accordingly, the present invention is intended to provide a calcium phosphate-based bioabsorbable microscrew and a method of manufacturing the same, wherein the calcium phosphate-based bioabsorbable microscrew is used to fix a barrier membrane in guided bone regeneration, which is performed to reinforce alveolar material absorbed into an implantation site and to repair a local bone defect, and includes a composite comprising hydroxyapatite and β-tricalcium phosphate, which are mixed, so that additional surgical removal is obviated after healing, a problem of a decreased repair rate due to the excessive metal fixed to a wounded portion and consequent overprotection thereof does not occur, and side effects are minimized.

### Technical Solution

Therefore, the present invention provides a calcium phosphate-based bioabsorbable microscrew, comprising hydroxyapatite and β-tricalcium phosphate. As such, the hydroxyapatite and the β-tricalcium phosphate may be mixed in amounts of 10 to 90 wt% and 10 to 90 wt%, respectively.

In addition, the present invention provides a method of manufacturing a calcium phosphate-based bioabsorbable microscrew, comprising: mixing a hydroxyapatite powder with a β-tricalcium phosphate powder, thus preparing a composite powder; adding and mixing the composite powder with a binder, thus preparing a screw-molding powder; molding the screw-molding powder using an injection-molding machine to produce a microscrew; primarily heat-treating the produced microscrew; and secondarily heat-treating the primarily heat-treated microscrew.

The hydroxyapatite powder and the β-tricalcium phosphate powder may be mixed in amounts of 10 to 90 wt% and 10 to 90 wt%, respectively, and the binder may include at least one selected from the group consisting of polyvinyl alcohol, polyethylene glycol, polystyrene, paraffin wax, stearic acid, eechenic acid, dibutyl phthalate, dioctyl phthalate, and ethylene vinyl acetate. The composite powder and the binder may be mixed in amounts of 20 to 80 wt% and 20 to 80 wt%, respectively.

Furthermore, molding using the injection-molding machine may be performed at 150 to 200°C, primarily heat-treating may be performed at 400 to 800°C, and secondarily heat-treating may be performed at 850 to 1180°C. The hydroxyapatite powder and the β-tricalcium phosphate powder may have a diameter of 0.1 to 200 µm.

Also, the method of the invention may further comprise subjecting the screw-molding powder to roll-milling at room temperature to form a plate, cooling the plate, and grinding the cooled plate into a powder, after preparing the screw-molding powder.

### Advantageous Effects

According to the present invention, the calcium phosphate-based bioabsorbable microscrew, manufactured using a composite comprising hydroxyapatite and β-tricalcium phosphate, which are mixed, has the effects of obviating additional surgical removal after healing, preventing a problem of a decreased repair rate due to the excessive metal fixed to a wounded portion and consequent overprotection, and minimizing side effects.

### Description of Drawings

FIG. 1 shows a process of manufacturing a microscrew according to the present invention;
FIG. 2 shows the microscrew manufactured by the process of the present invention; and
FIG. 3 shows the use of the microscrew according to the present invention.

### Best Mode

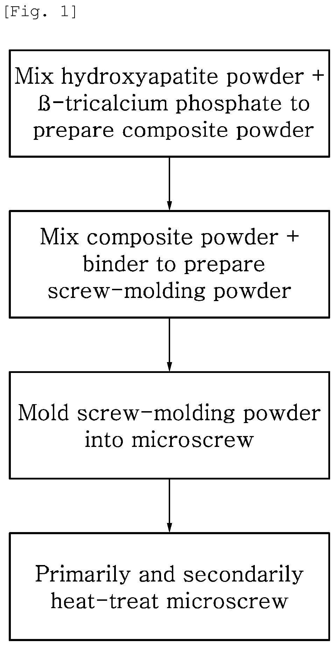

The present invention addresses a method of manufacturing a calcium phosphate-based bioabsorbable microscrew, as shown in FIG. 1, comprising:
a) mixing a hydroxyapatite powder with a β-tricalcium phosphate powder, thus preparing a composite powder;
b) adding and mixing the composite powder with a binder, thus preparing a screw-molding powder;
c) molding the screw-molding powder using an injection-molding machine to produce a microscrew;
d) primarily heat-treating the produced microscrew; and
e) secondarily heat-treating the primarily heat-treated microscrew.

In the method of manufacturing the calcium phosphate-based bioabsorbable microscrew according to the present invention, hydroxyapatite and β-tricalcium phosphate for forming a bioabsorbable microscrew are described below.

Hydroxyapatite functions as a support until new bone tissue maintains its structural stability, and β-tricalcium phosphate functions to increase a new osteoblast adhesion surface through ion exchange by rapid dissolution.

Hydroxyapatite has high biocompatibility, but is insoluble *in vivo* and thus impedes bone regeneration, which is undesirable. To solve this problem, β-tricalcium phosphate, which is biodegradable while being directly linked with bone, is used.

However, since β-tricalcium phosphate is quickly absorbed before bone regeneration, it is mixed at various ratios with hydroxyapatite, which is not biodegradable, thereby controlling biodegradability and absorbability. When 10 to 90 wt% of hydroxyapatite and 10 to 90 wt% of β-tricalcium phosphate are used, the formation of new bone within the bone defect may be promoted.

The use of hydroxyapatite in a larger amount than that of β-tricalcium phosphate can be found to promote the formation of new bone within the bone defect. Based on the test results of Table 1 below, mixing 10 to 90 wt% of hydroxyapatite with 10 to 90 wt% of β-tricalcium phosphate is evaluated to be very ideal to form a bone replacement.

**[Table 1]**

| | Component | | Action mechanism |
|---|---|---|---|
| | Hydroxyapatite | β-tricalcium phosphate | |
| 1 | 100 | 0 | Osteoconductivity |
| 2 | 0 | 100 | Absorption after dissolution |
| 3 | 90 | 10 | Osteoconductivity and absorption after dissolution |
| 4 | 60 | 40 | Osteoconductivity and absorption after dissolution |
| 5 | 40 | 60 | Osteoconductivity and absorption after partial dissolution |
| 6 | 10 | 90 | Osteoconductivity and absorption after dissolution |

The hydroxyapatite powder and the β-tricalcium phosphate powder preferably have a particle diameter of 0.1 to 200 µm. When the particle diameter is increased, the specific surface area increases, making it difficult to perform a kneading process. Hence, the powder size of both hydroxyapatite and β-tricalcium phosphate preferably falls in the range of 0.1 to 200 µm in diameter.

The composite powder (hydroxyapatite + β-tricalcium phosphate) at the above component ratio is added and mixed with the binder, thus preparing a screw-molding powder. The reason why the binder is used is described below.

The binder is a kind of resin or cement material, and functions to conglomerate particles so as to increase mechanical strength, bonding homogeneity, toughening, adhesion upon surface coating, and moldability.

Typically, a binder refers only to a binding material, but recently indicates a lubricant (for lubricating particles) or a plasticizer (a combination system including a material for making the binding material soft), as well as the binding material.

Currently, the above two meanings are used, but the latter meaning is more general.

Before the injection process, the binder used for the kneading (hydroxyapatite + β-tricalcium phosphate powder + binder) includes at least one selected from the group consisting of polyvinyl alcohol, polyethylene glycol, polystyrene, paraffin wax, stearic acid, eechenic acid, dibutyl phthalate, dioctyl phthalate, and ethylene vinyl acetate.

The binder is added and mixed with the composite powder (hydroxyapatite + β-tricalcium phosphate), thus preparing the screw-molding powder. Preferably, 20 to 80 wt% of the composite powder and 20 to 80 wt% of the binder are mixed.

If the amount of the binder exceeds 80 wt%, bonding homogeneity, adhesion upon surface coating, and moldability are increased but mechanical strength is decreased. On the other hand, if the amount thereof is less than 20 wt%, mechanical strength is increased but bonding homogeneity, adhesion upon surface coating, and moldability are decreased.

Then, the screw-molding powder thus obtained is molded into a microscrew, as shown in FIG. 1, using an injection-molding machine. As such, the temperature of the injection-molding machine in the molding process is preferably set to the range of 150 to 200°C.

If the temperature of the injection-molding machine is higher than 200°C, the binder may be carbonized. On the other hand, if the temperature thereof is lower than 150°C, moldability may decrease. Hence, the temperature of the injection-molding machine preferably falls in the range of 150 to 200°C.

The produced microscrew is primarily heat-treated. This is because oil and other impurities are removed from the surface of the microscrew.

The primary heat-treatment is preferably performed at a temperature of 400 to 800°C. If the temperature of the primary heat-treatment is lower than 400°C, oil and impurities are not completely removed. On the other hand, if the temperature thereof is higher than 800°C, excessive energy is wasted.

The primarily heat-treated microscrew is secondarily heat-treated. This is because the binder is removed from the microscrew. The secondary heat-treatment is preferably performed at a temperature of 850 to 1180°C.

If the temperature of the secondary heat-treatment is lower than 850°C, the binder is not completely removed from the microscrew and may be partially left behind on the microscrew, thus deteriorating the bioabsorbability of the microscrew and decreasing the density and strength of the product. On the other hand, if the temperature thereof is higher than 1180°C, tissue phase transformation of the microscrew may occur, resulting in low bioabsorbability. Hence, the secondary heat-treatment temperature of the microscrew preferably falls in the range of 850 to 1180°C.

Also, in the method of manufacturing the microscrew according to the present invention, the prepared screw-molding powder may be roll-milled at room temperature to form a plate, which is then cooled, and the cooled plate is ground into a powder, after which the powder is molded into a microscrew using an injection-molding machine.

The reason why the prepared screw-molding powder is roll-milled at room temperature to form a plate, which is then cooled and ground, is to ensure the stability of the mixture comprising hydroxyapatite, β-tricalcium phosphate and the binder.

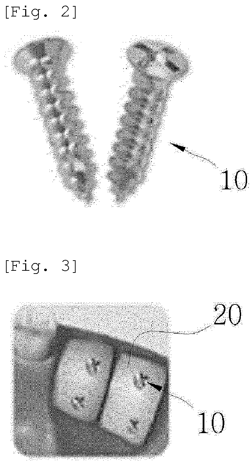

In the present invention, the calcium phosphate-based bioabsorbable microscrew thus manufactured is used as follows. Specifically, the corresponding portion of the gum to be bone grafted is incised, undergoes bone grafting, and is covered with a barrier membrane 20 as shown in FIG. 3, and the barrier membrane 20 is fixed with the microscrew 10 of the present invention.

A predetermined period of time after the barrier membrane is fixed with the microscrew of the present invention, the microscrew of the present invention is bioabsorbed, thus obviating additional surgical removal after healing, preventing a problem of a decreased repair rate due to the excessive metal fixed to a wounded portion and consequent overprotection, and generating no side effects.

## Claims

1. A calcium phosphate-based bioabsorbable microscrew, comprising hydroxyapatite and β-tricalcium phosphate.

2. The calcium phosphate-based bioabsorbable microscrew of claim 1, wherein the hydroxyapatite and the β-tricalcium phosphate are mixed in amounts of 10 to 90 wt% and 10 to 90 wt%, respectively.

3. A method of manufacturing a calcium phosphate-based bioabsorbable microscrew, comprising:
mixing a hydroxyapatite powder with a β-tricalcium phosphate powder, thus preparing a composite powder;
adding and mixing the composite powder with a binder, thus preparing a screw-molding powder;
molding the screw-molding powder using an injection-molding machine to produce a microscrew;
primarily heat-treating the produced microscrew; and
secondarily heat-treating the primarily heat-treated microscrew.

4. The method of claim 3, wherein the hydroxyapatite powder and the β-tricalcium phosphate powder are mixed in amounts of 10 to 90 wt% and 10 to 90 wt%, respectively.

5. The method of claim 3, wherein the binder comprises at least one selected from the group consisting of polyvinyl alcohol, polyethylene glycol, polystyrene, paraffin wax, stearic acid, eechenic acid, dibutyl phthalate, dioctyl phthalate, and ethylene vinyl acetate.

6. The method of claim 3 or 5, wherein the composite powder and the binder are mixed in amounts of 20 to 80 wt% and 20 to 80 wt%, respectively.

7. The method of claim 3, wherein the molding using the injection-molding machine is performed at 150 to 200°C.

8. The method of claim 3, wherein the primarily heat-treating is performed at 400 to 800°C.

9. The method of claim 3, wherein the secondarily heat-treating is performed at 850 to 1180°C.

10. The method of claim 3, wherein the hydroxyapatite powder and the β-tricalcium phosphate powder have a diameter of 0.1 to 200 µm.

11. The method of claim 3, further comprising subjecting the screw-molding powder to roll-milling at room temperature to form a plate, cooling the plate, and grinding the cooled plate into a powder, after the preparing the screw-molding powder.
